Europäisches Patentamt

European Patent Office

Office européen des brevets

(11) Publication number: **0 065 827**
**B1**

(19)

## EUROPEAN PATENT SPECIFICATION

(12)

(45) Date of publication of patent specification: **14.08.85**

(21) Application number: **82302209.0**

(22) Date of filing: **29.04.82**

(51) Int. Cl.⁴: **A 01 N 1/02,** A 61 L 17/00,
A 61 F 2/24

(54) Calcification resistant tissue for implantation.

(30) Priority: **30.04.81 US 259762**
**24.12.81 US 334107**

(43) Date of publication of application:
**01.12.82 Bulletin 82/48**

(45) Publication of the grant of the patent:
**14.08.85 Bulletin 85/33**

(84) Designated Contracting States:
**AT BE CH DE FR GB IT LI NL SE**

(56) References cited:
**FR-A-1 317 584**
**US-A-4 050 893**

(73) Proprietor: **EXTRACORPOREAL MEDICAL**
**SPECIALITIES INC.**
**Royal and Ross Road**
**King of Prussia Pennsylvania 19406 (US)**

(72) Inventor: **Lentz, David John**
**24811 Daphne East**
**Mission Viejo California 92691 (US)**
Inventor: **Pollock, Elisabeth Marie**
**20042 Royal Oak Court**
**Yorba Linda California 92686 (US)**

(74) Representative: **Jones, Alan John et al**
**CARPMAELS & RANSFORD 43 Bloomsbury**
**Square**
**London, WC1A 2RA (GB)**

Courier Press, Leamington Spa, England.

# 0 065 827

## Description

### Background of invention

This invention relates to the preparation of natural tissue for implantation, and more particularly, to the treatment of fixed tissue to inhibit mineralization, particularly calcification, upon implantation.

Animal tissues have in recent years found wide acceptance in the preparation of various prosthetic devices for use in humans. Most notable of these is the use of porcine heart valves to replace defective mitral, tricuspid and aortic valves in humans. Also of interest is the preparation of arteries, veins and human umbilical cords for use as arterial grafts and other tubular duct replacement in humans.

Porcine heart valves have been in use for several years with generally good results. The preparation of such valves for implantation is described in the literature and in the patent art as, for example, in U.S. Patents Nos. 3,966,401 and 4,050,893. Briefly, the valve is excised from the donor heart, trimmed and cleaned, and fixed by immersion in a tanning fluid such as a 0.2% glutaraldehyde solution. After several hours of treatment, the fixed valve is removed from the glutaraldehyde, rinsed, mounted on a stent, and stored in a glutaraldehyde solution until ready for use.

One problem which has been associated with the porcine heart valve in some individuals is calcification of the valve leaflets after an extended period of time resulting in reduced flexibility and eventual loss of efficiency in the operation of the valve. Significant calcification is readily visible in an X-ray of the affected valve.

It is accordingly an object of the present invention to provide a method to inhibit mineralization, and particularly calcification, of fixed natural tissue upon implantation.

It is a further object of this invention to provide a method for treatment of fixed porcine heart valve tissue to inhibit mineralization when used as a prosthetic valve replacement in humans.

It is a yet further object of this invention to provide fixed natural tissue characterized by improved resistance to calcification as compared to fixed natural tissue heretofore available.

These and other objects of the present invention will be apparent from the ensuing description and claims.

As used herein, the term "fixed" or "fixed tissue" refers to tissue which has been treated with a tanning solution such as 4% formaldehyde or 0.2% glutaraldehyde for a period of time and under conditions conventionally used to prepare natural tissue for implantation. The tanning process does not form any part of the present invention.

### Summary of invention

Natural tissue such as porcine heart valves or bovine pericardial tissue which has been fixed for implantation in accordance with conventional procedures is treated prior to implantation with a aqueous solution of a water soluble salt of a sulfated higher aliphatic alcohol containing from 7 to 18 carbon atoms such as sodium dodecyl sulfate (SDS). The treatment may be effected in a 1% solution of SDS at ambient temperatures and for a period of about 24 hours. The treated tissue is removed from the SDS solution, rinsed, and returned to storage in sterile glutaraldehyde until needed for implantation.

Porcine heart valve cusp tissue treated in accordance with the present invention is characterized in that the tissue evidences an average of less than 1.0 micrograms $Ca^{++}$ per milligram wet tissue when explanted after 8 weeks residence in the abdominal wall muscle of a male Sprague-Dawley rat. Bovine pericardium tissue is similarly characterized in evidencing an average of less than 2.0 micrograms $Ca^{++}$ per milligram wet tissue when explanted after 8 weeks.

### Description of preferred embodiments

Natural tissue in accordance with the present invention is characterized by an exceptionally low rate of calcification when implanted in a living animal. Tissue calcification is generally observed whenever fixed natural tissue is implanted in muscle tissue or placed in the blood stream of a warm blooded animal. While the tissue of the present invention is directed primarily for use in the replacement of defective heart valves in humans, the propensity of the tissue to undergo calcification is readily determined by animal tests.

In accordance with the method for treating natural tissue to reduce its propensity to undergo calcification, fixed natural tissue is treated with an aqueous solution of a water soluble salt of a sulfated higher aliphatic alcohol containing from 7 to 18 carbon atoms. The alkyl unit may be straight chain or branched, and preferred alkyl sulfates include the water soluble salts of lauryl sulfate, myristyl sulfate, cetyl sulfate, stearyl, sulfate, and oleyl sulfate. Mixtures of two or more alkyl sulfates may also be used if desired. The salt of the alkyl sulfate is preferably soluble in water at room temperature to a concentration of at least 2%, and preferably at least 5% by weight. Suitable salts include the sodium, potassium and ammonium salts of $C_{7-18}$ alkyl sulfate, and amine salts such as triethylamine-lauryl sulfate. The sodium salt of lauryl (dodecyl) surface is most particularly preferred and its use is illustrated in the following detailed example.

An SDS treatment solution (1%) was prepared by dissolving 10 g of SDS and 1.4 g of N-2-hydroxyethylpiperazine-N'-2-ethanesulfonic acid (HEPES) in one liter of distilled water, and the pH of the solution was adjusted to 7.4 with 1.0 N sodium hydroxide.

Fifty pieces of fixed porcine valve cusp tissue weighing from about 20 to 30 mg each were rinsed in saline solution to remove the glutaraldehyde, then placed in 50 ml of the SDS treatment solution at ambient

2

temperature of 20—25°C. The solution containing the tissue pieces was stirred continuously on a magnetic stirrer. The treatment solution was changed after 8 hours, and the treatment was continued for a total of 24 hours.

After the SDS treatment and in preparation for implantation, the tissue pieces were rinsed in 0.2% glutaraldehyde solution, resterilized by immersion for 24 hours in a solution of 1% glutaraldehyde/20% isopropyl alcohol, and returned to sterile 0.2% glutaraldehyde solution to await implantation.

The effectiveness of the SDS treatment in retarding calcification of the fixed tissue was determined by animal implant studies according to the following procedure.

Male Sprague-Dawley rats weighing 180—200 g were anesthetized and prepared for abdominal surgery under sterile conditions. The abdominal area was shaved, disinfected, and a lengthwise midline skin incision approximately 4 cm long was made in the ventral surface. The skin was separated from the underlying muscle, and three small pouches were formed in the muscle on either side of the midline incision by a small incision followed by blunt dissection of the abdominal muscle wall. One piece of SDS-treated tissue, rinsed in sterile saline to remove the glutaraldehyde, was inserted in each muscle pouch. The skin incision was closed and the animal returned to its cage. Implantations were made in 10 rats for a total of 60 pieces of SDS-treated tissue. A control group of 10 rats were implanted under identical conditions with a total of 60 pieces of fixed porcine valve cusp tissue not subjected to the SDS treatment.

Five rats from the control group and five from the SDS test group were sacrificed after four weeks and the implanted tissue examined for calcification by X-ray and hydrochloric acid extraction. The entire abdominal wall with implants *in situ* was excised and X-rayed. The implants were then removed and two set aside for histological examination while the remaining four were dissected free of surrounding tissue and extracted individually in 5 ml of 0.6 N HCl at 70°C for 96 hours. The extract solution was then assayed for calcium by atomic absorption.

The remaining rats in both the test and control groups were sacrificed after 8 weeks and the extent of calcification of the implanted tissue determined as above.

The results of the animal study are presented in Table I.

TABLE I
Degree of calcification

| Porcine cusp tissue | 4 Weeks | | 8 Weeks | |
|---|---|---|---|---|
| | X-ray[2] | Extraction[1] | X-ray[2] | Extraction[1] |
| SDS-treated Rat 1 | 0/6 | 0.0 | 0/6 | 0.37 |
| 2 | 0/6 | 0.0 | 0/6 | 0.45 |
| 3 | 0/6 | 0.0 | 0/6 | 1.21 |
| 4 | 0/6 | 0.0 | 0/6 | 0.20 |
| 5 | 0/6 | 0.0 | 0/6 | 0.39 |
| AVG. | | 0.0 | | 0.52 |
| Control | | | | |
| Rat 1 | 5/6 | 19.07[3] | 6/6 | 2.84 |
| 2 | 6/6 | 4.61 | 6/6 | 12.91 |
| 3 | 6/6 | 5.09 | 6/6 | 4.03 |
| 4 | 5/6 | 10.76 | 6/6 | 6.49 |
| 5 | 4/6 | 8.66 | 6/6 | 3.66 |
| AVG. | | 9.63 | | 5.99 |

[1] Average of 4 values, μg $Ca^{++}$/mg wet wt. tissue
[2] Evaluated by visual examination, 4/6=4 of 6 samples evidenced a significant degree of calcification.
[3] Average of 2 values in 4 week data for Control.

3

As illustrated by the data in Table I, porcine valve cusp tissue treated with the SDS solution remained substantially free of calcification for a period of 8 weeks under the severe calcification conditions inherent in the rat test. The correlation between calcification in the rat test and human experience is such that the extensive calcification detected in the rat control group after 8 weeks would not be expected to occur in humans until after several years exposure. The SDS treatment would accordingly be expected to retard calcification in humans for an additional period of years beyond that normally experienced prior to the onset of calcification.

The procedure described above is one that has produced good results, but it will be apparent to those skilled in the art that many variations in this procedure are posible. For example, the concentration of the SDS treatment solution may range from 0.1 to 5.0% or higher, and other water soluble salts of $C_{7-18}$ alkyl sulfates may be substituted for the SDS. Treatment temperatures may range from 5°C to 50°C; and treatment times may range from 4 hours to several days.

The pH of the treatment solution is preferably from neutral to slightly basic and may range from 4.0 to 9.0, and most preferably from 7.0 to 7.5. Buffering agents other than HEPES may be selected according to the desired pH value. In addition, other ingredients both active and inactive may be utilized in combination with the alkyl sulfate in the treatment solution. Such variations may be developed by those skilled in the art with little or no experimentation to suit individual desires.

The animal tests described above were repeated to determine the calcification characteristics of fixed porcine heart valve cusp tissue and bovine pericardial tissue treated with a one percent (1%) solution of SDS for periods ranging from 1 to 7 days, and implanted for periods ranging from 4 weeks to 24 weeks. The solution was prepared as described above.

A control study was made with similar tissue treated with a one percent (1%) solution of leuco-toluidine blue, a treatment suggested in the art as being useful to inhibit calcification of fixed natural tissue during implantation. The treatment solution was prepared according to the method disclosed in U.S.—A—2,571,593. The solution was prepared in one liter distilled water which had been previously boiled and purged with $CO_2$ to remove dissolved air. While continuing the $CO_2$ purge, there was added 10 g toluidine blue, 7.4 g sodium hydrosulfite and 2 cc concentrated HCl. The pH of the solution was about 3. The continer was sealed and shaken until the blue color disappeared and a muddy grey precipitate formed. After standing for 24 hours at room temperature, aliquots of the supernatant liquid were repeatedly shaken with activated charcoal and filtered until a clear solution was obtained. The solution was maintained in a stoppered bottle under $CO_2$ atmosphere until ready for use.

The tissue treatment procedure was the same for both the SDS and the leuco-toluidine blue (LTD) solutions. Approximately 50 pieces of fixed tissue, each weighing 20—30 mg were placed in a container with 50 ml of the treatment solution. The solutions were maintained at ambient temperatures and were not changed for the duration of the treatment period. Samples of tissue were removed after 1, 3, 5 and 7 days and prepared for implantation as previously described.

The results of comparative testing of SDS and leucotoluidine blue in the Sprague-Dawley rat test for calcification of fixed porcine valve cusp tissue and fixed bovine pericardial tissue over a period of 4 to 24 weeks implantation are presented in Tables II and III. These data clearly illustrate the remarkable results obtained with the SDS treatment to inhibit calcification. The fixed natural tissue so treated is unique in its ability to withstand calcification during implantation. Moreover, the physical properties of the tissue such as tensile strength, fatigue resistance and flexibility are not affected by the treatment and the performance of the tissue in a prosthetic device is not impaired.

## TABLE II
### Calcification of implanted natural tissue[1]

Duration of implantation

| Length of treatment | 4 Weeks | | 8 Weeks | | 12 Weeks | |
|---|---|---|---|---|---|---|
| | SDS | LTD | SDS | LTD | SDS | LTD |
| **A. Porcine valve cusp Tissue** | | | | | | |
| 1 day | 0.27±0.73 | 3.0±4.1 | 0.0±0.0 | 10.57±6.62 | 1.33±0.08 | 19.87±8.77 |
| 3 days | 0.0±0.0 | 1.87±2.27 | 0.02±0.03 | 7.96±8.60 | 0.77±0.16 | 16.86±8.30 |
| 5 days | 0.0±0.0 | 10.58±6.64 | N/T | 7.76±9.52 | 0.48±0.06 | 12.20±12.25 |
| 7 days | N/T | N/T | 0.005±0.018 | 2.46±3.39 | 8.02±6.33 | 6.57±0.80 |
| **B. Bovine pericardial tissue** | | | | | | |
| 1 day | 0.12±0.56 | 5.2±6.8 | 1.47±3.75 | 56.56±40.11 | 3.99±5.02 | 78.39±55.00 |
| 3 days | 0.64±2.86 | 18.3±5.9 | 0.62±2.72 | 80.27±27.20 | 0.22±0.06 | 113.50±56.39 |
| 5 days | 0.53±0.14 | 2.7±5.5 | 1.26±3.41 | 4.20±4.90 | 0.05±0.05 | 5.84±6.03 |
| 7 days | 1.66±1.87 | 14.7±11.2 | 0.22±0.95 | 9.90±8.20 | 0.33±1.48 | 3.18±3.31 |

[1] Calcification data reported as $\mu g$ $Ca^{++}$/mg wet tissue, based on 20 samples.
N/T Not Tested

**0 065 827**

TABLE III
Long term calcification of implanted natural tissue[1]

| Duration of implantation | Treatment solution | |
|---|---|---|
| | SDS | LTD |
| 16 weeks | 0.10±0.05 | 9.01±5.39 |
| 20 weeks | 0.42±0.04 | 23.26±24.77 |
| 24 weeks | 0.63±2.40 | 24.48±18.38 |

[1] Porcine valve cusp tissue, treated for 7 days with SDS or LTD solution, implanted in Sprague-Dawley rats; calcification data $\mu$g $Ca^{++}$/mg wet tissue, based on 20 samples.

Due to the inherent variability in natural tissue samples and in test animals, the results of the calcification test in Sprague Dawley rats are subject to considerable variability as evidenced by the data presented in Tables II and III. Thus, it is necessary to replicate the test to establish meaningful results and, for purposes of defining the tissue of the present invention, we have selected calcification values based on an average of 20 samples. Specifically, porcine heart valve tissue and bovine pericardial tissue in accordance with the present invention are defined in terms of maximum and preferred degrees of calcification after 4 to 12 weeks in the Sprague-Dawley rat test based on 20 samples as follows:

| | Duration of implantation | Degree of calcification $\mu$g $Ca^{++}$/mg tissue | |
|---|---|---|---|
| | | Maximum | Preferred |
| A. Porcine heart valves | 4 weeks | 0.5 | <0.1 |
| | 8 weeks | 1.0 | <0.2 |
| | 12 weeks | 3.0 | <1.0 |
| B. Bovine pericardia | 4 weeks | 2.0 | <1.0 |
| | 8 weeks | 2.5 | <1.0 |
| | 12 weeks | 3.0 | <1.0 |

Fixed natural tissue having resistance to calcification comparable to the tissues of the present invention have not heretofore been known. Tissue treated with leucotoluidine blue, while demonstrating some short-term advantage over untreated tissue, falls far short of the tissue of the present invention. Accordingly, the present invention provides a new and improved product representing a substantial advance in state-of-the-art relating to implantable devices.

Although the proceeding description has been limited to porcine heart valves and bovine pericardia, the invention also includes veins, arteries and other tissues intended for implantation in humans which are derived from animals or humans. Accordingly, the present invention is not intended to be limited to the specific tissues of the examples presented herein.

## Claims

1. A method for inhibiting the mineralization of fixed natural tissue intended for implantation in a living body comprising contacting fixed natural tissue intended for implantation with an aqueous solution of water soluble salt of $C_{7-18}$ alkyl sulfate.

2. The method of Claim 1 wherein the water soluble salt is a sodium, potassium, ammonium or amine salt.

3. The method of Claim 1 or Claim 2 wherein said $C_{7-18}$ alkyl sulfate is lauryl, myristyl, cetyl, stearyl or oleyl sulfate.

4. The method of any one of Claims 1 to 3 wherein the concentration of said salt of $C_{7-18}$ alkyl sulfate in said solution is from 0.1 to 5% by weight.

6

5. The method of Claim 1 wherein said aqueous solution comprises from 0.1 to 5% by weight sodium dodecyl sulfate.

6. The method of any one of Claims 1 to 5 wherein the fixed tissue is a porcine heart valve.

7. Fixed natural tissue suitable for implantation in a living body, characterized in that a piece of said tissue remains substantially free of calcification after 4 weeks when implanted in the abdominal wall muscle of a male Sprague-Dawley rat.

8. Fixed natural tissue suitable for implantation in a living body, characterized in that a piece of said tissue evidences less than 1.0 micrograms $Ca^{++}$ per milligram wet tissue after 8 weeks when implanted in the abdominal wall muscle of a male Sprague-Dawley rat.

9. Fixed natural tissue suitable for implantation in a living body, characterized in that a piece of said tissue evidences less than 3.0 micrograms $Ca^{++}$ per milligram wet tissue after 12 weeks when implanted in the abdominal wall muscle of a male Sprague-Dawley rat.

10. A porcine heart valve suitable for implantation in a living body, characterized in that a piece of cusp tissue excised from said valve remains substantially free of calcification for at least 4 weeks when implanted in the abdominal wall muscle of a male Sprague-Dawley rat.

**Patentansprüche**

1. Ein Verfahren zur Verhinderung der Mineralisation eines zur Implantation in einem lebenden Körper vorgesehenen fixierten natürlichen Gewebes, umfassend das Inberührungbringen des zur Implantation vorgesehenen fixierten natürlichen Gewebes mit einer wässerigen Lösung eines wasserlöslichen Salzes eines $C_{7-18}$-Alkylsulfats.

2. Das Verfahren nach Anspruch 1, worin das wasserlösliche Salz ein Natrium-, Kalium-, Ammonium- oder Aminsalz ist.

3. Das Verfahren nach Anspruch 1 oder 2, worin das genannte $C_{7-18}$-Alkylsulfat Lauryl-, Myristyl-, Cetyl-, Stearyl- oder Oleylsulfat ist.

4. Das Verfahren nach einem der Ansprüche 1 bis 3, worin die Konzentration des genannten Salzes des $C_{7-18}$-Alkylsulfats in der genannten Lösung von 0,1 bis 5 Gew.-% beträgt.

5. Das Verfahren nach Anspruch 1, worin die genannte wässerige Lösung von 0,1 bis 5 Gew.-% Natriumdodecylsulfat enthält.

6. Das Verfahren nach einem der Ansprüche 1 bis 5, worin das fixierte Gewebe eine Schweineherzklappe ist.

7. Zur Implantation in einem lebenden Körper geeignetes fixiertes natürliches Gewebe, dadurch gekennzeichnet, daß ein Stück des genannten Gewebes nach vierwöchiger Implantation im Bauchdeckenmuskel einer männlichen Sprague-Dawley-Ratte im wesentlichen frei von Verkalkung bleibt.

8. Zur Implantation in einem lebenden Körper geeignetes fixiertes natürliches Gewebe, dadurch gekennzeichnet, daß ein Stück dieses Gewebes nach achtwöchiger Implantation im Bauchdeckenmuskel einer männlichen Sprague-Dawley-Ratte weniger als 1,0 µg $Ca^{++}$ je mg nasses Gewebe aufweist.

9. Zur Implantation in einem lebenden Körper geeignetes fixiertes natürliches Gewebe, dadurch gekennzeichnet, daß ein Stück des genannten Gewebes nach zwölfwöchiger Implantation im Bauchdeckenmuskel einer männlichen Sprague-Dawley-Ratte weniger als 3,0 µg $Ca^{++}$ je mg nasses Gewebe aufweist.

10. Zur Implantation in einem lebenden Körper geeignete Schweineherzkappe, dadurch gekennzeichnet, daß ein aus der genannten Klappe ausgeschnittenes Stück von Spitzengewebe im wesentlichen frei von Verkalkung für wenigstems vier Wochen bleibt, wenn es in den Bauchdeckenmuskel einer männlichen Sprague-Dawley-Ratte implantiert ist.

**Revendications**

1. Procédé pour inhiber la minéralisation des tissus naturels fixés destinés à être implantés dans un corps vivant, comprenant la mise en contact du tissus naturel fixé destiné à être implanté avec une solution aqueuse d'un sel alkyl(en $C_{7-18}$)-sulfate hydrosoluble.

2. Procédé selon la revendication 1, dans lequel le sel hydrosoluble est un sel de sodium, de potassium, d'ammonium ou d'amine.

3. Procédé selon la revendication 1 ou la revendication 2, dans lequel le dit sel alkyl(en $C_{7-18}$)-sulfate est un lauryl-, myristyl-, cétyl-, stéaryl- ou oléylsulfate.

4. Procédé selon l'une quelconque des revendications 1 à 3, dans lequel la concentration du dit sel alkyl-(en $C_{7-18}$)-sulfate dans la dite solution est de 0,1 à 5% en poids.

5. Procédé selon la revendication 1, dans lequel la dite solution aqueuse comprend de 0,1 à 5% en poids de dodécylsulfate de sodium.

6. Procédé selon l'une quelconque des revendications 1 à 5, dans lequel le tissu fixé est une valvule de coeur de porc.

7. Tissu naturel fixé utilisable pour être implanté dans un corps vivant, caractérisé en ce qu'un morceau du dit tissu reste sensiblement exempt de calcification après 4 semaines d'implantation dans le muscle de la paroi abdominale d'un rat Sprague-Dawley mâle.

8. Tissu naturel fixé utilisable pour être implanté dans un corps vivant, caractérisé en ce qu'un morceau du dit tissu présente moins de 1,0 microgrammes de Ca$^{++}$ par milligramme de tissu humide après 8 semaines d'implantation dans le muscle de la paroi abdominale d'un rate Sprague Dawley mâle.

9. Tissue naturel fixé utilisable pour être implanté dans un corps vivant, caractérisé en ce qu'un morceau du dit tissu présente moins de 3,0 microgrammés de Ca$^{++}$ par milligramme de tissu humide après 12 semaines d'implantation dans le muscle de la paroi abdominale d'un rat Sprague Dawley mêle.

10. Valvule de coeur de porc utilisable pour être implantée dans un corps vivant, caractérisée en ce qu'un morceau de tissu de cuspide excisé de la dite valve reste sensiblement exempt de calcification pendant au moins 4 semaines d'implantation dans le muscle de la paroi abdominale d'un rat Sprague-Dawley mâle.